# EUROPEAN PATENT APPLICATION

(11) **EP 2 184 016 A1**
(43) Date of publication of application: **12.05.2010**
(21) Application number: 08828788.3
(22) Date of filing: 27.08.2008
(51) Int. Cl.: A61B 17/06

(54) **MEDICAL SUTURING NEEDLE**

(30) Priority: 27.08.2007 JP 2007219169
(71) Applicant: MANI Inc., Utsunomiya-shi, Tochigi 321-3231 (JP)
(72) Inventor: MATSUTANI, Kanji, Utsunomiya-shi Tochigi 321-3231 (JP)
(74) Representative: Göhring, Robert
(86) International application number: PCT/JP2008/065309
(87) International publication number: WO 2009/028557

(57) **Abstract**

Provided is a medical suturing needle or a round needle having a circular section, which is balanced between the magnitude of an insertion resistance to living tissues and the difficulty in damaging gloves, thereby to improve the safety. The medical suturing needle (A) comprises a ball-shaped needle tip (1), a tapered portion (2) continuing to the needle tip (1), and a trunk portion (3) continuing to the tapered portion (2). The ball of the needle tip (1) has a diameter of 0.25 mm to 0.34 mm, and the tapered portion (2) has a tapering angle of 8.5 degrees to 9.4 degrees.

## Description

### Technical Field

The present invention relates to medical suturing needles, and in particular, to a medical suturing needle that does not penetrate through a surgical glove so as not to inj ure the operator by balancing the insertion resistance in inserting through the living tissues and the resistance in penetrating through the surgical glove worn by the operator.

### Background Art

The medical suturing needle used to suture the living tissues includes a needle called a round needle in which the cross-sectional shape from the needle tip portion to the trunk portion is circular and the cross-sectional shape of the trunk portion is circular, triangular, square, flat-shape, or the like; and a needle called a cutting needle having a polygonal shape including a triangle and in which a cutting edge is formed at a plurality of ridge lines. The round needle includes a blunt needle in which the distal end is formed blunt so as to pass through while widening the tissue after inserting the needle tip to the living tissue; whereas the cutting needle passes through while cutting open the tissues. Thus, the optimum medical suturing needle is selectively used in correspondence to the property of the site to be sutured and other conditions.

When suturing the affected area, the largest resistance (insertion resistance) generally generates when inserting the needle tip through the living tissue, and the resistance tends to lower thereafter. Thus, in order to smoothly proceed with the surgery while alleviating the work of the doctor, development is being carried out with regards to how to have sharper needle tip and how to reduce the insertion resistance.

### Disclosure of The Invention

However, with reduction in insertion resistance, problems such as easily penetrating through the surgical glove worn by the operator including doctors and nurses at the time of the operation, and easily injuring the operator arise. In particular, if the surgical glove is damaged by the medical suturing needle attached with the body fluid of the patient, the operator wearing such surgical glove may be infected.

It is an object of the present invention to provide a medical suturing needle or a round needle having a circular cross-section from the needle tip portion to the trunk portion (having tapered portion), particularly the blunt needle in which the distal end is formed blunt, in which the magnitude of an insertion resistance to living tissues and the difficulty in damaging gloves are balanced.

To solve the above problems, the present inventors conducted various experiments focusing on the shape of the needle tip of the round needle (blunt needle) and the tapered portion from the needle tip to the trunk portion. In the experiments, the needle tip was formed to a ball-shape and the diameter of the ball was changed, and the tapering angle from the needle tip to the trunk portion was also changed to create samples of the medical suturing needle, and the insertion resistance with respect to the surgical glove actually worn by the operator and the inserting material (product name: pole bear) having properties close to the living tissues were measured with the samples. The "tapering angle" referred herein refers to an included angle of the extended lines that face each other when the opposing tapered surfaces are extended in the needle tip direction with the maximum diameter of the ball of the needle tip (diameter when virtual circle is drawn at the needle tip) as a reference point.

The size of the ball diameter of the needle tip has been debated over the balance between the magnitude of the insertion resistance of the blunt needle and the difficulty in damaging the gloves. In other words, in the case of the blunt needle (round needle), the size of the ball is important since the largest insertion resistance applies when the needle tip end passes through the tissue (wall thereof), and the tapered portion (angle) on the back part side of the ball is not a problem. In particular, since the blunt needle is conventionally used to suture soft tissues of the liver and the like, the tapering angle is not considered to influence the insertion resistance since the resistance rapidly lowers after becoming a maximum when passing through the thin epidermis of the tissue with the needle tip end. However, recently, the shape of the distal end etc. of the blunt needle is being reviewed with the use of the blunt needle for suturing thicker and harder tissues such as diaphragm and other internal organs other than the liver. The inventors thus measured the insertion resistance using the pole bear (close to skin or thick and hard tissues), which was not used in the measurement of the insertion resistance of the blunt needle up to now, and found that the tapering angle also influences the insertion resistance other than the size of the ball diameter of the needle tip. The suturing needles having various tapering angles are prepared, and the experiment was conducted.

In conducting the experiment, the magnitude of the insertion resistance that can be tolerated at the time of suturing is hearing investigated in advance from doctors who actually performs the suturing operation and set as an insertability target value, and the magnitude of the insertion resistance with respect to the surgical glove is set in correspondence to the target value as a resistance target value, and whether or not the medical suturing needle that satisfies both target values can be realized is determined.

As a result of the experiment, a medical suturing needle according to the present invention is a medical suturing needle including a ball-shaped needle tip, a tapered portion continuing to the needle tip, and a trunk portion continuing to the tapered portion; where the ball of the needle tip has a diameter in a range of between greater than or equal to 0.25 mm and smaller than or equal to 0.34 mm, and the tapered portion has a tapering angle in a range of between greater than or equal to 8.5 degrees and smaller than or equal to 9.4 degrees.

The medical suturing needle (hereinafter referred to as "suturing needle") of the present invention has an insertion resistance of an extent tolerated by doctors and has small possibility of damaging gloves worn by the operator. In other words, the insertion resistance with respect to the living tissue and the insertion when penetrating the glove can be balanced. Thus, a suturing needle having satisfactory insertability in which safety is ensured can be obtained.

### Brief Description of The Drawings

Fig. 1 is a view describing a configuration of a suturing needle according to the present example.
Fig. 2 is a view illustrating the result of an insertion experiment with respect to a pole bear.
Fig. 3 is a view illustrating the result of an insertion experiment with respect to a surgical glove.

### Description of Reference Numerals

- A: suturing needle
- 1: needle tip
- 2: tapered portion
- 3: trunk portion
- 4: proximal end
- 5: stop-hole

### Best Mode For Carrying Out The Invention

A preferred embodiment of a suturing needle according to the present invention will be described below. The suturing needle according to the present invention ensures safety by reducing the possibility of damaging the operator at the time of the operation and also ensures the insertability to the living tissues of an extent that can be tolerated by the operator. In other words, not only the reduction of the insertion resistance to the living tissues is focused, but also a balance with the safety of the operator is achieved.

In the present invention, the suturing needle merely needs to be a round needle having a tapered portion continuing to the needle tip, and the cross-sectional shape of the trunk portion or the entire shape are not particularly limited. In other words, a curved needle curved at a curvature radius set in advance, a linear needle of substantially linear shape, or the like is preferably selected in correspondence with the affected area to be sutured for the suturing needle of the present invention.

The round needle having the tapered portion, that is, having a circular cross-sectional shape from the needle tip to the trunk portion is not used to cut the muscle or the tissue such as the skin and pass therethrough to suture, but is generally used to insert the needle tip to the epidermis and then pass through while widening the tissue to suture.

In the present invention, the diameter (range of between 0.25 mm and 0.34 mm) of a ball configuring the needle tip is obtained from the results of the experiment. The tapering angle (tapering angle is in range of between 8.5 degrees and 9.4 degrees) of the tapered portion continuing to the needle tip is also obtained from the results of the experiment. In particular, the thickness of the trunk portion of the suturing needle is standardized, and set in a range of between about 0.07 mm and about 1.4 mm.

The material for forming the suturing needle is not particularly limited, and steel such as piano wire and martensitic stainless steel, in which hardness can be obtained by heat treatment, austenitic stainless steel, and the like can be used. However, rust may produce in the steel and the martensitic stainless steel at the circulation stage. Thus, although hardening by heat treatment cannot be expected, the austenitic stainless steel is preferably used taking into consideration that workability is satisfactory and rust does not produce.

### First example

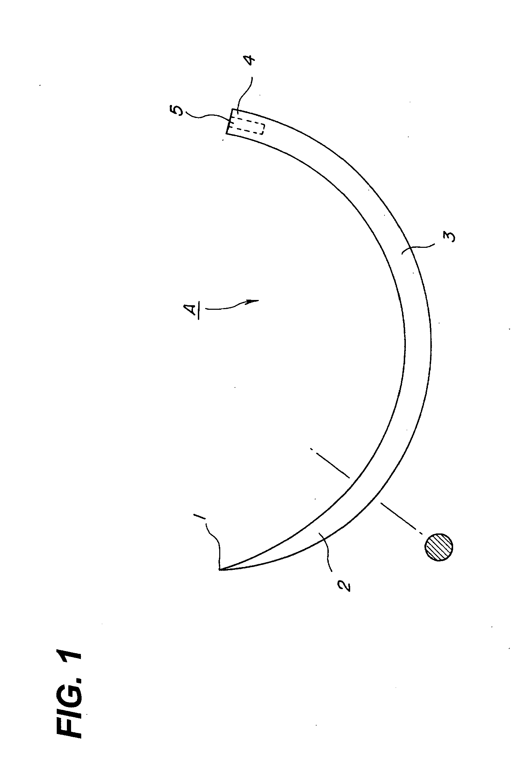

An example of the suturing needle according to the present invention will be described using drawings. Fig. 1 is a view describing a configuration of a suturing needle according to the present example.

A suturing needle A illustrated in Fig. 1 has a needle tip 1 formed at the distal end, a tapered portion 2 formed continuing to the needle tip 1, a trunkportion 3 formed continuing to the tapered portion 2, and an proximal end 4 with a stop-hole 5 for attaching the suturing thread (not illustrated) formed at the end of the trunk portion 3. The cross-sectional shapes of the tapered portion 2 and the trunk portion 3 in the suturing needle A are circular.

In particular, the needle tip 1 is formed to a ball-shape, where the diameter of the ball configuring the needle tip 1 is set in a range of between 0.25 mm and 0.34 mm. The tapering angle of the tapered portion 2 is set in a range of between 8.5 degrees and 9.4 degrees. The suturing needle A ensuring insertability of an extent tolerable by the operator and sufficient safety can be achieved by including the ball-shaped needle tip 1 and the tapered portion 2 in such numerical ranges.

The suturing needle A uses a material that exhibits work hardening by performing a cold wire drawing process on the austenitic stainless steel wire at a surface reduction rate set in advance and that has the austenitic tissue stretched to a fiber-form. The suturing needle A is configured as a curved needle called a 1/2 circle.

In the present invention, the diameter of the ball configuring the needle tip and the tapering angle of the tapered portion are obtained from the results of experiments. Therefore, the tapering angle of the tapered portion can be set by appropriately setting the length (length of tapered portion) from the trunk portion to the needle tip if the diameter of the ball of the needle tip is set. In other words, the length of the tapered portion is not constant in all suturing needles, and changes according to the thickness.

The experiment conducted to obtain the suturing needle A according to the present invention will now be specifically described.

In this experiment, the tolerable magnitude of the insertion resistance (insertability target value, Newton, N) was set by carrying out hearing investigation from doctors in advance, and the magnitude of the insertion resistance (resistance target value, N) when penetrating the surgical glove was set at the same time, where such set values were assumed as the target value of the insertion resistance when inserting the pole bear and the target value of the insertion resistance when penetrating the surgical glove.

The insertability target value was 2.9 N and the resistance target value was 1.5 N.

The test pieces were such that the thickness of the trunk portion 3 is 1.28 mm (constant), and the diameter of the ball configuring the needle tip 1 is 0.2 mm, 0.3 mm, 0.4 mm, 0.5 mm, and 0. 6 mm. The tapered portion 2 had the length set to integral multiples (9D, 12D, 13D, 17D) of the thickness D, and the portion between the needle tip 1 and the trunk portion 3 formed to a tapered shape in a range of the set length. Therefore, in such test pieces, the tapering angle differs if the diameter of the needle tip 1 differs even if the length of the tapered portion 2 is the same.

A plurality of (ten) test pieces in which the length of the tapered portion 2 differs for every diameter of the needle tip 1 was fabricated, and the insertion resistance was measured by performing insertion with respect to the thickness of 1.1 mm of the pole bear and the insertion with respect to the surgical glove for five test pieces each.

Therefore, if the diameter of the needle tip 1 is set to 0.2 mm, four types of lengths of the tapered portion 2 exist and ten test pieces were fabricated for each length, and hence the inserting experiment was conducted 40 times. Since five types are set for the diameter of the needle tip 1, the inserting experiment was conducted 200 times.

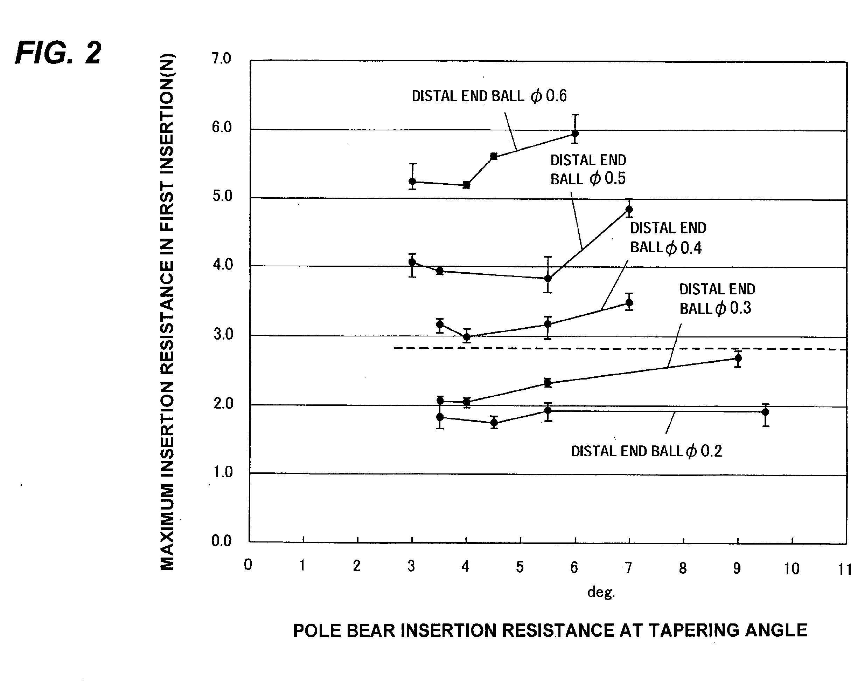

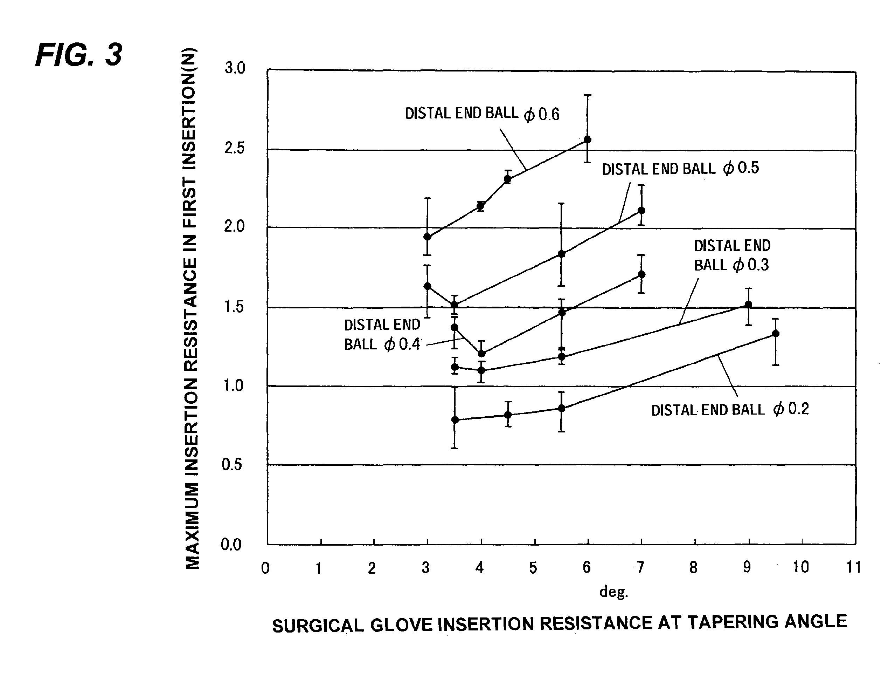

The result of the insertion experiment with respect to the pole bear is illustrated in Fig. 2. The result of the inserting experiment with respect to the surgical glove is illustrated in Fig. 3. In Figs. 2 and 3, the vertical axis illustrates the maximum insertion resistance value (N), illustrating the data obtained by the first insertion experiment in each test piece. The horizontal axis illustrates the tapering angle of the tapered portion 2, illustrating the actual measurement value of each test piece. The dotted line in Fig. 2 illustrates the insertability target value, and the dotted line in Fig. 3 illustrates the resistance target value.

As illustrated in Figs. 2 and 3, it is apparent from the result of the present experiment that the insertion resistance tends to become smaller the smaller the diameter of the ball of the needle tip 1, and the insertion resistance tends to become smaller the smaller the tapering angle of the tapered portion 2. The results of the insertion experiment by the test piece of each condition will now be reviewed using Figs. 2 and 3.

In the case of the test piece in which the diameter of the ball of the needle tip 1 is 0.2 mm, the insertion resistance value with respect to the pole bear was about 2 N, and the insertion resistance was the smallest. The insertion resistance with respect to the surgical glove was about 0.7 N when the tapering angle was about 3.5 degrees, rose as the tapering angle became larger, and was about 1.3 N when the tapering angle was about 9.5 degrees.

In the case of such test piece, the insertability with respect to the living tissues is satisfactory, but the surgical glove tends to be easily damaged. The test piece in which the tapering angle is about 9.5 degrees can be used without problem.

In the case of the test piece in which the diameter of the ball of the needle tip 1 is 0.3 mm, the insertion resistance value with respect to the pole bear was about 2 N when the tapering angle was about 3.5 degrees, and was about 2.8 N when the tapering angle was about 9 degrees. The insertion resistance value of such test piece is the closest to the insertability target value. The insertion resistance with respect to the surgical glove was about 1.1 N when the tapering angle was about 3.5 degrees, rose as the tapering angle became larger, and was about 1.5 N when the tapering angle was about 9 degrees.

In the case of such test piece, the insertion resistance value slightly below the insertability target value is indicated, and the insertion resistance value substantially the same as the resistance target value is indicated when the tapering angle of the tapered portion 2 is about 9 degrees. Therefore, the insertion resistance of when inserting to the living tissues and the resistance of when penetrating the surgical glove worn by the operator are well balanced.

However, the insertion resistance value with respect to the surgical glove is smaller than the resistance target value and the surgical glove may be easily damaged when the tapering angle was between about 3.5 degrees and 5.5 degrees, and thus this parameter cannot be used.

In the case of the test piece in which the diameter of the ball of the needle tip 1 is 0.4 mm, the insertion resistance value with respect to the pole bear was about 3 N when the tapering angle was about 4 degrees, was about 3.2 N when the tapering angle was about 5. 5 degrees, and was about 3. 5 N when the tapering angle was about 7 degrees. The insertion resistance with respect to the surgical glove was about 1.2 N when the tapering angle was about 4 degrees, rose as the tapering angle became larger, and was about 1.5 N when the tapering angle was about 5. 5 degrees and about 1.7 N when the tapering angle was about 7 degrees.

In the case of such test piece, the insertion resistance value with respect to the pole bear is greater than the insertability target value regardless of the value of the tapering angle. Thus, degradation in the workability may occur when doctors perform suturing. If the tapering angle of the tapered portion 2 is about 4 degrees, the insertion resistance value with respect to the surgical glove becomes lower than the resistance target value, thereby easily damaging the glove, and thus this parameter cannot be used.

However, when the tapering angle is about 5.5 degrees, the insertion resistance value with respect to the pole bear is slightly greater than the insertability target value and the insertion resistance value with respect to the surgical glove substantially matches the resistance target value. Thus, this parameter can be used without problem.

In the case of the test piece in which the diameter of the ball of the needle tip 1 is 0.5 mm, the insertion resistance value with respect to the pole bear was about 4 N when the tapering angle was about 3.5 degrees, was about 3.8 N when the tapering angle was about 5. 5 degrees, and was about 4.8 N when the tapering angle was about 7 degrees. The insertion resistance with respect to the surgical glove was about 1.5 N when the tapering angle was about 3.5 degrees, rose as the tapering angle became larger, and was about 2.2 N when the tapering angle was about 7 degrees.

In the case of such test piece, the insertion resistance value with respect to the pole bear is greater than the insertability target value regardless of the value of the tapering angle. Thus, degradation of the workability may occur when doctors perform suturing. The insertion resistance value with respect to the surgical glove matches the resistance target value when the tapering angle of the tapered portion 2 is about 3. 5 degrees, but this parameter cannot be used from the standpoint of the workability of the doctor.

In the case of the test piece in which the diameter of the ball of the needle tip 1 is 0.6 mm, the insertion resistance value with respect to the pole bear was about 5.2 N when the tapering angle was about 4 degrees, and was about 5.9 N when the tapering angle was about 6 degrees. The insertion resistance with respect to the surgical glove was about 1. 9 N when the tapering angle was about 3 degrees, and was about 2. 6 N when the tapering angle was about 6 degrees.

In the case of such test piece, the insertion resistance value with respect to the pole bear is greater than the insertability target value regardless of the value of the tapering angle, and the insertion resistance value with respect to the surgical glove is also greater than the resistance target value. Thus, although the possibility of damaging the surgical glove reduces, degradation of the workability may occur when doctors perform suturing, and thus this parameter cannot be used.

As a result of the reviews made above, the suturing needle A has a diameter of the ball configuring the needle tip 1 in the range of between 0.25 mm and 0.34 mm, and the tapering angle of the tapered portion 2 in the range of between 8.5 degrees and 9.4 degrees. In such suturing needle A, the work of the doctors may slightly increase but is within a tolerable range when suturing the affected area, and the possibility of damaging the surgical glove can be made extremely small.

The above example has been described for the suturing needle in which the cross-sectional shape of the trunk portion 3 is circular, but it is not limited to circular, and the suturing needle having a cross-sectional shape of a triangle, square, flat shape, and the like can be obviously used. In particular, the bending force and the gripping force become important when suturing tissues harder than the liver tissues, where blunt needle has been conventionally used, such as the diaphragm.

In this case, the bending force is preferably made stronger by having the cross-sectional shape of the trunk portion 3 to a triangle, a square, and the like, and the gripping force is preferably enhanced by arranging a groove on a surface to be gripped by a needle forceps. In particular, if the cross-sectional shape of the trunk portion 3 is triangular, two or more projections are formed on the surface opposite to the peak, the groove is formed between the projections, and the extended line of a perpendicular line with respect to the surface in the grove intersects one part of the peak, the needle satisfactorily engages with the needle forceps, whereby the gripping force can be greatly enhanced.

### Industrial Applicability

The suturing needle according to the present invention enables operators including doctors and nurses to safely carry out the suturing operation, and is advantageous when used for the operation of suturing the living tissues.

## Claims

1. A medical suturing needle comprising a ball-shaped needle tip, a tapered portion continuing to the needle tip, and a trunk portion continuing to the tapered portion, wherein
the ball of the needle tip has a diameter in a range of between greater than or equal to 0.25 mm and smaller than or equal to 0.34 mm, and the tapered portion has a tapering angle in a range of between greater than or equal to 8.5 degrees and smaller than or equal to 9.4 degrees.

2. The medical suturing needle according to claim 1, wherein cross-sectional shapes of the tapered portion and the trunk portion are formed to a circle, and the trunk portion has a thickness in a range of between greater than or equal to 0.7 mm and smaller than or equal to 1.4 mm.
